# NOUVEAU FASCICULE DE BREVET EUROPEEN

(11) **EP 1 968 711 B2**
(45) Date de publication et mention de la décision concernant l'opposition: **22.02.2023**
(45) Mention de la délivrance du brevet: 10.04.2013
(21) Numéro de dépôt: 07712641.5
(22) Date de dépôt: 05.01.2007
(51) Int. Cl.: A61K 8/34, A61K 8/73, A61Q 19/08, A61K 9/00, A61K 47/10

(54) **GEL VISCOELASTIQUE A USAGE DERMATOLOGIQUE**
VISKOELASTISCHES DERMATOLOGISCHES GEL
DERMATOLOGICAL VISCOELASTIC GEL

(30) Priorité: 06.01.2006 FR 0600138
(43) Date de publication de la demande: 17.09.2008
(73) Titulaire: Anteis SA, 1228 Plan-Les-Ouates, Genève (CH)
(72) Inventeur: BOS, Gilles, CH-1228 Plan-Les-Ouates (CH)
(74) Mandataire: Ricker, Mathias
(86) Numéro de dépôt international: PCT/FR2007/000016
(87) Numéro de publication internationale: WO 2007/077399

(56) Documents cités:
- WO-A1-2004/073759
- US-A- 5 106 615
- US-A- 5 106 615
- US-A1- 2004 185 021
- US-A1- 2004 185 021
- Hege Bothne Wik and al.: "Rheology of hyaluronan" In: "The Chemistry, Biology and Medical Applications of Hyaluronan and its Derivatives", 1998, Portland Press Ltd, London ISBN: 1 85578 119 0 vol. 72, pages 25-32,
- Wikipedia "Autoclave"
- ASHP Guidelines 1993

## Description

L'invention concerne un gel viscoélastique à usage dermatologique.

Divers polymères d'origine naturelle tels que le collagène, l'acide hyaluronique, ou les dérivés cellulosiques, sont fréquemment utilisés en médecine esthétique et en dermatologie, pour le comblement des rides, le remodelage du visage, l'augmentation du volume des lèvres, et le rajeunissement de la peau du visage; ce dernier type de traitement est directement dérivé de la mésothérapie.

Pour le rajeunissement de la peau, les praticiens utilisent fréquemment de l'acide hyaluronique, quelquefois en association avec des complexes de vitamines, d'acides aminés, de sels minéraux et d'acides nucléiques.

La présente invention a pour base l'association d'un polysaccharide d'origine naturelle, qui est l'acide hyaluronique, et d'un alcool visqueux, qui est le glycérol, afin de proposer une composition injectable prête à l'emploi qui optimise le traitement de rajeunissement de la peau. La présente invention fournit un gel de polysaccharide d'origine naturelle utilisé en injection en dermatologie selon la revendication 1 ou 2.

On a en effet trouvé que l'association d'acide hyaluronique et d'une faible quantité d'un alcool visqueux biocompatible, à savoir le glycérol, donne une composition dont la viscosité augmente fortement et que lorsque l'alcool visqueux biocompatible est de plus hydrophile, il augmente considérablement les propriétés de rétention de l'eau, lorsque la composition est injectée sous la peau à traiter.

Par d'alcool biocompatible visqueux et fortement hydrophile, on entend tout alcool biocompatible visqueux ayant une forte affinité pour l'eau. Dans un alcool, plus la densité de fonctions OH est élevée, plus il est hydrophile. Selon l'invention, l'alcool biocompatible visqueux et fortement hydrophile est le glycérol, un alcool de petit poids moléculaire (92,09 g/mol) et contenant trois fonctions OH, qui est extrêmement hydrophile.

Il a été trouvé que l'addition d'une faible quantité, à savoir de 0,5 à 5 % poids/volume, d'un alcool visqueux biocompatible entraîne une augmentation importante de la viscosité d'une solution de polysaccharide d'origine naturelle à 0,1 - 5% poids/volume, stabilise cette solution pendant la stérilisation et maintient des propriétés visqueuses particulièrement intéressantes pour le rajeunissement du tissu cutané. L'alcool visqueux peut participer à la restructuration de la peau et à la maturation des cellules de ce tissu et assure l'isotonicité du mélange.

Selon l'invention, on utilise un alcool biocompatible ayant des propriétés antiseptiques, ce qui réduit ce risque d'infections cutanées. Cet alcool est le glycérol. En effet ce risque n'est pas négligeable lors d'un traitement de rajeunissement compte tenu du nombre important d'injections pratiquées dans le visage.

L'acide hyaluronique se trouve dans le commerce à différentes masses moléculaires (MW) et à différentes concentrations selon le fabricant. Selon l'invention, on utilise une concentration de 1,8 % poids/volume pour de l'acide hyaluronique de poids faible à moyen (0,5 à 1,8 MDa) et une concentration de 1,5 % poids/volume pour de l'acide hyaluronique de poids élevé (2,0 à 3,0 MDa).

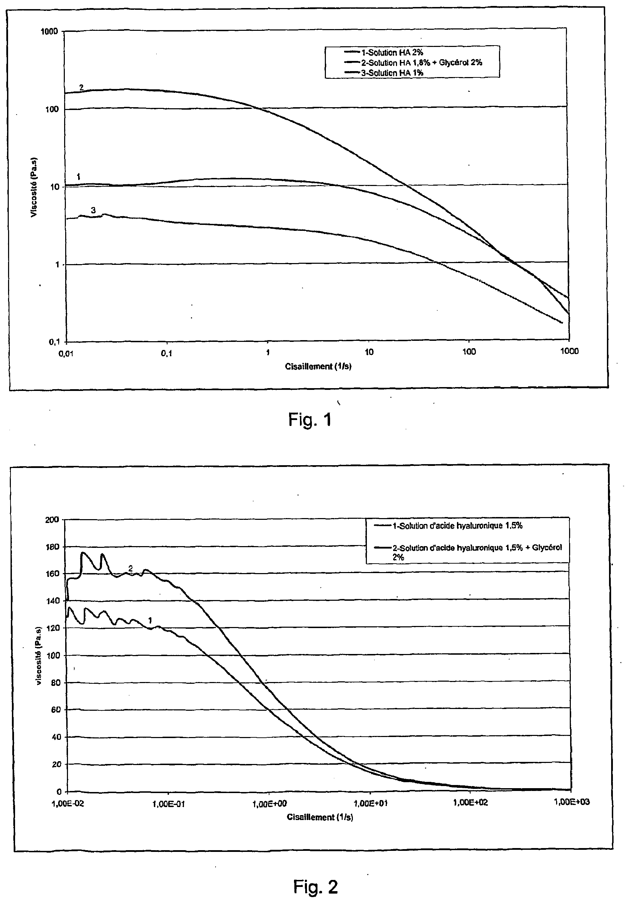

Les figures 1 à 3 sont des graphiques représentant les viscosités des compositions des exemples 1 et 2 selon l'invention et de l'exemple 3 (comparatif).

### Exemple 1: Influence de la concentration en acide hyaluronique et de la présence de glycérol sur la viscosité de solutions pour le rajeunissement du visage

On a préparé trois solutions à base d'un même acide hyaluronique caractérisé par un poids moléculaire moyen de 1,6 MDa.

La première solution est une solution d'acide hyaluronique à 1 %.

La seconde solution est une solution du même acide hyaluronique, mais concentré à 2 %.

La troisième solution ne contient que 1,8 % de ce même acide hyaluronique auquel a été ajouté du glycérol à 2% poids/volume.

Les trois préparations ont été stérilisées par chaleur humide puis leurs propriétés rhéologiques ont été analysées à l'aide d'un rhéomètre, en mesurant la viscosité en fonction du taux de cisaillement imposé au produit.

Il apparaît clairement que, d'après le graphique de la figure 1, pour les faibles taux de cisaillement (correspondant à ceux auxquels est exposée la préparation pour le rajeunissement du tissu cutané après injection), l'adjonction de glycérol à une solution d'acide hyaluronique a plus d'influence pour l'obtention d'une préparation de haute viscosité que l'augmentation de la concentration en acide hyaluronique.

### Exemple 2: Influence du glycérol sur la viscosité de solutions d'acide hyaluronique de haut poids moléculaire

On a préparé deux solutions à base d'acide hyaluronique caractérisé par un poids moléculaire moyen très élevé (2,6 MDa).

La première préparation est une solution d'acide hyaluronique à 1,5 %.

La seconde préparation contient également 1,5 % d'acide hyaluronique de haut poids moléculaire auquel a été ajouté du glycérol à 2% poids/volume. Les deux préparations ont été stérilisées par chaleur humide puis leurs propriétés rhéologiques ont été analysées à l'aide d'un rhéomètre, en mesurant la viscosité en fonction du taux de cisaillement imposé au produit.

Le graphique de la figure 2 met en évidence que, même lorsque la préparation est composée d'un acide hyaluronique de haut poids moléculaire -donc initialement caractérisée par une viscosité élevée-, le glycérol tend à augmenter tout de même les propriétés visqueuses du produit.

### Exemple 3: Stabilisation de la préparation par le glycérol lors de la stérilisation (comparatif)

On a préparé une solution à 1,5 % d'acide hyaluronique caractérisé par un poids moléculaire moyen très élevé (2,6 MDa). On a ensuite stérilisé cette solution par chaleur humide (préparation 1).

Du glycérol a été ajouté à quelques millilitres de cette solution stérilisée (préparation 2).

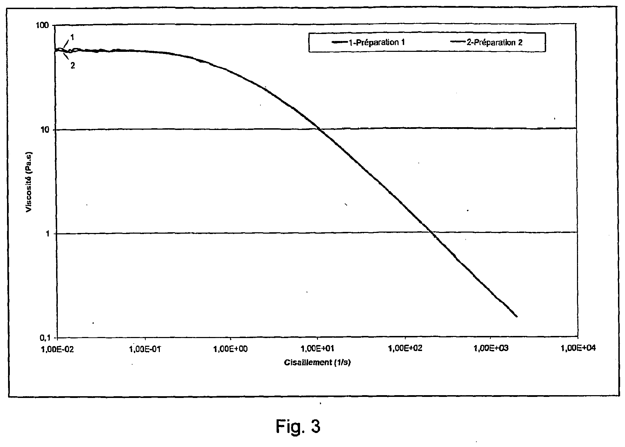

Aucune différence rhéologique n'est observée entre ces deux préparations ; le graphique de la figure 3 et les exemples précédemment présentés mettent en évidence l'effet stabilisant du glycérol lors de la stérilisation.

Il est donc essentiel que l'alcool visqueux soit mélangé à la solution d'acide hyaluronique avant la stérilisation pour obtenir l'augmentation de la viscosité.

Après stérilisation, la composition peut être mise sur une forme prête à l'emploi, par exemple dans une ampoule ou une fiole contenant la dose à injecter par l'intermédiaire d'une seringue.

La composition peut comprendre les adjuvants usuels en dermatologie, ajoutés au mélange avant la stérilisation. De tels adjuvants sont les vitamines, les acides minéraux, les sels minéraux et les acides nucléiques.

## Revendications

1. Gel de polysaccharide d'origine naturelle utilisé en injection en dermatologie, **caractérisé en ce qu'**il comprend une solution aqueuse contenant 1,8 % poids/volume d'acide hyaluronique de MW moyen (0,5 à 1,8 MDa) et 2 % poids/volume de glycérol, obtenue par préparation d'une solution aqueuse de l'acide hyaluronique et du glycérol puis stérilisation de cette solution par la chaleur humide, et éventuellement les adjuvants usuels en dermatologie, la stérilisation par la chaleur humide après mélange des constituants ayant pour effet d'augmenter sensiblement la viscosité du gel résultant.

2. Gel de polysaccharide d'origine naturelle utilisé en injection en dermatologie, **caractérisé en ce qu'**il comprend une solution aqueuse contenant 1,5 % poids/volume d'acide hyaluronique de MW élevé (2,0 à 3,0 MDa) et 2 % poids/volume de glycérol, obtenue par préparation d'une solution aqueuse de l'acide hyaluronique et du glycérol puis stérilisation de cette solution par la chaleur humide, et éventuellement les adjuvants usuels en dermatologie, la stérilisation par la chaleur humide après mélange des constituants ayant pour effet d'augmenter sensiblement la viscosité du gel résultant.

## Patentansprüche

1. Natürliches Polysaccharidgel, das zur Injektion in der Dermatologie verwendet wird, **dadurch gekennzeichnet, dass** es eine wässrige Lösung, die 1,8 Gew./Vol.-% Hyaluronsäure mit mittlerem Molekulargewicht (0,5 bis 1,8 MDa) und 2 Gew./Vol.-% Glycerol enthält, die durch Herstellung einer wässrigen Lösung der Hyaluronsäure und des Glycerols und danach Sterilisation dieser Lösung durch feuchte Wärme hergestellt wird, und eventuell die in der Dermatologie üblichen Zusatzstoffe umfasst, wobei die Sterilisation durch feuchte Wärme nach Mischen der Bestandteile eine deutliche Erhöhung der Viskosität des erhaltenen Gels zur Folge hat.

2. Natürliches Polysaccharidgel, das zur Injektion in der Dermatologie verwendet wird, **dadurch gekennzeichnet, dass** es eine wässrige Lösung, die 1,5 Gew./Vol.-% Hyaluronsäure mit hohem Molekulargewicht (2,0 bis 3,0 MDa) und 2 Gew./Vol.-% Glycerol enthält, die durch Herstellung einer wässrigen Lösung der Hyaluronsäure und des Glycerols und danach Sterilisation dieser Lösung durch feuchte Wärme hergestellt wird, und eventuell die in der Dermatologie üblichen Zusatzstoffe umfasst, wobei die Sterilisation durch feuchte Wärme nach Mischen der Bestandteile eine deutliche Erhöhung der Viskosität des erhaltenen Gels zur Folge hat.

## Claims

1. A polysaccharide gel of natural origin used in injection in dermatology, **characterized in that** it comprises an aqueous solution containing 1.8% by weight/volume of hyaluronic acid with an average MW (0.5 to 1.8 MDa) and 2% by weight/volume of glycerol, obtained by preparing an aqueous solution of the hyaluronic acid and glycerol and then by sterilizing the solution with moist heat, and optionally usual adjuvants in dermatology, the sterilization with moist heat after mixing the constituents having the effect of substantially increasing the viscosity of the resulting gel.

2. A polysaccharide gel of natural origin used in injection in dermatology, **characterized in that** it comprises an aqueous solution containing 1.5% by weight/volume of hyaluronic acid with high MW (2.0 to 3.0 MDa) and 2% by weight/volume of glycerol, obtained by preparing an aqueous solution of the hyaluronic acid and glycerol and then by sterilizing the solution with moist heat, and optionally usual adjuvants in dermatology, the sterilization with moist heat after mixing the constituents having the effect of substantially increasing the viscosity of the resulting gel.
